# EUROPEAN PATENT SPECIFICATION

(11) **EP 2 613 850 B1**
(45) Date of publication and mention of the grant of the patent: **23.11.2016**
(21) Application number: 11710153.5
(22) Date of filing: 11.03.2011
(51) Int. Cl.: A61Q 11/00, A61K 8/19, A61K 8/25, A61K 8/29, A61K 8/02, C09C 1/36

(54) **COMPOSITE PARTICLE ACTIVE AND METHOD FOR MAKING THE SAME**
ZUSAMMENGESETZTER PARTIKELWIRKSTOFF UND HERSTELLUNGSVERFAHREN DAFÜR
PARTICULES COMPOSITES ACTIVES ET LEUR PROCÉDÉ DE PRODUCTION

(30) Priority: 10.09.2010 WO PCT/CN2010/001396
(43) Date of publication of application: 17.07.2013
(73) Proprietor: Unilever PLC, London EC4Y 0DY (GB); Unilever N.V., 3013 AL Rotterdam (NL)
(72) Inventor: DENG, Yan, Shanghai 200335 (CN); DING, Guanjun, Shanghai 200050 (CN); LI, Xiaoke, Shanghai 200335 (CN)
(74) Representative: Whaley, Christopher
(86) International application number: PCT/EP2011/053740
(87) International publication number: WO 2012/031785

(56) References cited:
- WO-A1-97/32934
- WO-A1-2009/024372
- JP-A- 2003 275 600
- US-A- 5 858 333
- US-A1- 2001 051 136
- US-A1- 2006 115 437

## Description

### FIELD OF THE INVENTION

The present invention is directed to a composite particle active and method for making the composite particle. More particularly, the invention is directed to composite particle active suitable for use in the mouth to, at the very least, aid in the remineralization and whitening of teeth by interacting with phosphate ions and delivering calcium and phosphate sources to the teeth of consumers. The particles, therefore, are suitable for use in compositions that are used in the mouth. Such particles can, for example, be used in a toothpaste or gel composition, mouthwash, as well as chewing gum compositions. Moreover, the composite particle active of this invention comprises a first component core suitable to immediately improve an oral care characteristic and a second component coating that is suitable to interact with phosphate ions to produce calcium and phosphate *in situ* reaction products that surprisingly adhere well to teeth to provide immediate and long term benefits.

### BACKGROUND OF THE INVENTION

Many products we consume have a negative impact on our teeth and mouth. Acidic drinks and sweets, for example, can result in tooth erosion by attacking enamel that coats and protects the teeth. Such an attack on enamel often, and undesirably, causes hypersensitivity of the teeth. Moreover, foods and beverages we consume, like tomato sauce, berries, beets, soda or pop, coffee and tea can stain teeth and thereby result in a smile that is not bright and white. Tobacco based products and certain medications can also lead to teeth that look yellow or even brown. Of course, teeth that are not healthy can lead to bad breath and the growth of unwanted bacteria in the mouth.

Products that address tooth decay and/or whitening have been developed. Such products often comprise peroxides, abrasives or both in order to clean and whiten teeth. These types of products are often not desired since they do not contribute to the remineralization of teeth and can cause damage to teeth and gums if overused. Products comprising calcium sources have been developed in an attempt to enhance the characteristics of teeth. Such products, however, do not positively adhere to teeth and thereby may only contact teeth for a brief period prior to being rinsed out of the mouth in wash water.

It is of increasing interest to develop products that provide immediate and long term benefits to teeth. Moreover, it is of increasing interest to develop composite particle actives that can benefit teeth when supplied from a multitude of formats, like toothpaste or gel compositions, mouthwashes as well as chewing gum compositions. This invention, therefore, is directed to composite particle actives that are suitable to deliver calcium and phosphate sources to teeth for a long enough period of time to ensure excellent whitening results. The invention is also directed to a method for making the same. The composite particle actives of the present invention comprises a first component core for immediately improving an oral care characteristic and a second component coating that is suitable to interact with phosphate ions to produce calcium and phosphate *in situ* reaction products that surprisingly adhere well to teeth for both immediate and long term benefits.

### ADDITIONAL INFORMATION

Efforts have been disclosed for making oral care products. In WO 2008/068149 A1 and WO 2008/068248 A1, oral care products with calcium and phosphate are described.

Other efforts have been disclosed for making oral care products. In U.S. Patent Nos. 4,083,955, 5,605,675 and 6,214,321 B1, processes and compositions for remineralizing dental enamel are described.

Still other efforts have been disclosed for making oral care products. In U.S. Patent Nos. 6,365,132 B1 and 5,735,942, compositions for use on teeth are described.

In U.S. Patent Application Nos. 2002/0064504 A1 and 2009/0264291 A1, additional efforts have been disclosed for making oral care compositions. The former describes dental anti-hypersensitivity compositions and the latter describes compositions and methods for preventing or reducing plaque and/or gingivitis using a dentifrice with a bioactive glass.

None of the additional information above describes a composite particle active comprising a core and coating as described herein. Moreover, none of the additional information describes a method for making such a particle as described herein.

### SUMMARY OF THE INVENTION

In a first aspect, the present invention is directed to a composite particle active comprising:
(a) a first component core for improving an oral care characteristic wherein the first component core comprises silica, titanium dioxide, zinc oxide, mica, calcium carbonate, barium sulfate, or a mixture thereof; and
(b) a second component coating that interacts with phosphate ions to produce a calcium and phosphate *in situ* reaction product that adheres to tooth enamel, dentin or both and that is a precursor for hydroxyapatite formation and wherein the second component coating comprises calcium gluconate, calcium oxide, calcium lactate, calcium hydroxide, calcium sulfate, calcium carboxymethyl cellulose, calcium alginate, calcium salts of citric acid, calcium silicate or a mixture thereof;
Wherein at least 70 to 100% of the outer surface area of the first component core is coated with second component coating.

In a second aspect, the present invention is directed to a method for making the composite particle active of the first aspect of this invention.

All other aspects of the present invention will more readily become apparent upon considering the detailed description and examples which follow.

Soluble and insoluble, as used herein, refers to the solubility of a source (e.g., like calcium) in water. Soluble means a source that dissolves in water to give a solution with a concentration of at least 0.1 moles per liter at room temperature. Insoluble means a source that dissolves in water to give a solution with a concentration of less than 0.001 moles per liter at room temperature. Slightly soluble, therefore, is defined to mean a source that dissolves in water to give a solution with a concentration of greater than 0.001 moles per liter at room temperature and less than 0.1 moles per liter at room temperature. Oral care composition means a composition suitable for use in the mouth and for veterinary and/or human applications but especially for use in applications for the human mouth. Benefitting teeth means at the very least whitening, remineralization or desensitizing teeth or decreasing bacteria within the mouth where the decreasing of bacteria can be the result of employing an antimicrobial agent and the desensitizing of teeth is the result of composite particle active interacting with phosphate ions found in the mouth and/or within the oral care composition and adhering to the teeth to, for example, yield new hydroxyapatite formation on dentinal tubules. Diameter is meant to mean the longest distance measurable in the event the composite particle active is not a perfect sphere. Remineralization, as used herein, means the *in situ* generation of hydroxyapatite on teeth to reduce the likelihood of tooth decay and improve the appearance of teeth by whitening through the generation of such new hydroxyapatite. Such remineralization also results in whitening of teeth, all of which can include results from the adhering of composite particle active to the teeth and/or the formation of amorphous calcium phosphate on teeth. Single-phase composition means a one phase composition having both calcium and phosphate sources therein and prior to dispensing or unpackaging and use. Anhydrous, as used herein, means substantially free of water (e.g., no water to less than 5% by weight water and preferably less than 1% by weight water). Dual-phase product means having required and complementary calcium and phosphate hydroxyapatite precursors in separate compositions and stored in separate compartments. Composition as used herein includes, for example, paste, powder, gel, liquid (like mouthwash), spray, foam, balm, a composition carried on a mouthstrip or a buccal adhesive patch, chewable tablet (or pastille), lozenge, cream, beverage or a strip of gum. Preferably, however, the composition is a paste like toothpaste or a gel for teeth. Carrier, as used herein, means a component in the composition other than the composite particle active and phosphate source whereby the carrier is suitable to deliver the active and any phosphate source present therein. First component core, as used herein, is meant to include the portion of the composite particle active that is coated whereby the core can comprise particle or an aggregate of particle like, for example, an agglomerate of TiO₂. Core is also meant to include a component that provides immediate (e.g., within three (3) uses of the composition, but preferably, within 1 second to 5 minutes of using) whitening benefits to teeth via physical mechanisms. Long term benefit is meant to include a benefit that will last at least for weeks, but preferably, at least four (4) months. In general, the core is meant to be a component that may improve a characteristic of teeth. Second component coating, as used herein, means a coating that forms an external coat or clad on at least a portion of the first component core. Not to be bound by theory, adhering, as used herein, can include effectively depositing and/or bonding or "sticking" to teeth as a result of calcium in the coating of the composite particle active and phosphate ion interactions. Coating is also meant to include a component that provides benefits to teeth over time through biological or chemical mechanisms like the formation of hydroxyapatite. Composite particle active, therefore, is meant to mean a particle that may provide both immediate and long term benefits to teeth after use. *In situ* reaction product means a product comprising calcium and phosphate generated in the mouth.

All ranges defined herein are meant to include all ranges subsumed therein unless specifically stated otherwise. Comprising, as used herein, is meant to include consisting essentially of and consisting of, and should be understood to not preclude the inclusion of other components.

### BRIEF DESCRIPTION OF THE DRAWINGS

The subject matter which is regarded as the invention is particularly pointed out and distinctly claimed in the concluding portion of the specification. The invention, however, may be best understood by reference to the following description taken in conjunction with the accompanying drawing figures in which:
Figure 1 (A and B) shows composite particle active adhesion to enamel after one treatment.
Figure 2 (A and B) shows composite particle active adhesion to enamel after fourteen treatments.
Figure 3 shows composite particle active adhesion to dentin after one treatment.
Figure 4 shows composite particle active adhesion to dentin after fourteen treatments.
Figure 5 (A and B) shows conventional silicone and composition particle active deposition on the surface of teeth;
Figure 6 (a-d) shows Transmission Election Microscopy Images of uncoated and coated titanium dioxide particles; and
Figure 7 shows results from a consumer tooth whitening study after 2 weeks and 4 weeks treatment as compared to baseline.

### DETAILED DESCRIPTION OF THE PREFERRED EMBODIMENTS

The only limitation with respect to the composite particle active that may be used in this invention is that the same is suitable for use in the mouth. Typically, the core of the composite particle active comprises a material suitable to physically and immediately improve characteristics of teeth, and especially, whiten teeth. Such a material typically has an index of refraction from 1.2 to 3, preferably, from 1.3 to 2.8, and most preferably, from 1.5 to 2.8, including all ranges subsumed therein. Such core materials that are suitable to physically improve teeth comprise silica, titanium dioxide, zinc oxide, mica (including coated mica like commercially available iron oxide coated mica), calcium carbonate, barium sulfate or a mixture thereof. The core of the composite particle active typically makes up from 3 to 98%, and preferably, from 6 to 65%, and most preferably, from 10 to 55% by weight of the composite particle active, based on total weight of the composite particle active and including all ranges subsumed therein. In an especially preferred embodiment, the core is at least 50% by weight titanium dioxide or zinc oxide, and most preferably, from 60 to 100% by weight titanium dioxide or zinc oxide, based on total weight of the first component core.

The coating suitable to adhere to tooth enamel, dentin or both typically comprises a metal element such as calcium, and optionally, other metals like potassium, sodium, aluminum, magnesium, as well as mixtures thereof. In a preferred embodiment, the same is suitable to provide a biological or chemical improvement to teeth which is both long and short term (e.g., results in whitening and hydroxyapatite formation). Preferably, the coating employed comprises at least 50% by weight elemental calcium, and most preferably, at least 65% by weight elemental calcium based on total weight of metal in the coating. In an especially preferred embodiment, the metal element in the coating is from 80 to 100% by weight elemental calcium, based on total weight of metal in the second component coating and including all ranges subsumed therein. In another especially preferred embodiment, the core and the coating are slightly soluble or insoluble in water, but most preferably, insoluble in water.

70 to 100% of the outer surface area of the first component core is coated with the second component coating.

The diameter of the composite particle active is often from 10 nm to less than 50 microns, and preferably, from 75 nm to less than 10 microns. In an especially preferred embodiment, the diameter of composite particle active is from 500 nm to 5 microns, including all ranges subsumed therein. In yet another especially preferred embodiment, at least 40%, and preferably, at least 60%, and most preferably, from 75 to 99.5% of the diameter of the composite particle active is the result of core, including all ranges subsumed therein.

An oral care composition suitable to carry the composite particle active of this invention usually comprises from 0.25 to 40%, and preferably, from 0.5 to 20%, and most preferably, from 0.5 to 15% by weight composite particle active, based on total weight of the oral care composition and including all ranges subsumed therein. In an especially preferred embodiment, the composition comprises an overall metal (e.g., calcium ion) content of less than 40%, and preferably, less than 30%, and most preferably, from 1 to 25% by weight metal element, based on total weight of the composition and including all ranges subsumed therein.

In still another especially preferred embodiment, composite particle active may be added to a composition along with an additional metal source like a calcium source. Such an additional metal source may be identical to that which is used to coat the core of the composite particle active (e.g., calcium silicate or calcium oxide). When added, the additional metal source typically makes up from 0.1 to 35%, and preferably, from 1 to 25%, and most preferably, from 10 to 20% by weight of the total weight of the oral care composition and including all ranges subsumed therein.

The second component coating comprises calcium gluconate, calcium oxide, calcium lactate, calcium hydroxide, calcium sulfate, calcium carboxymethyl cellulose, calcium alginate, calcium salts of citric acid, calcium silicate, mixtures thereof or the like. In another desired embodiment, the calcium source in the coating will comprise calcium silicate.

These compounds are also suitable to include as reagents for calcium sources when making the composite particle active of this invention. In a preferred embodiment, the reagent suitable for *in situ* generation of coating material comprises calcium silicate, calcium phosphate, calcium gluconate, calcium oxide, potassium chloride, calcium lactate, calcium carbonate, calcium hydroxide, calcium sulfate, calcium carboxymethyl cellulose, calcium chloride, magnesium chloride, aluminum chloride, calcium alginate, calcium salts of citric acid, mixtures thereof or the like.

Similar salts with sodium, magnesium, aluminum and potassium in lieu of calcium may, for example, be used as reagents to provide the anionic portion of the second component coating during particle manufacturing. In a preferred embodiment, the reagent suitable for *in situ* generation of coating material comprises silica, sodium silicate, potassium silicate, sodium alginate, sodium sulfate, tetraethyl orthosilicate, or a mixture thereof.

In yet another preferred embodiment, the coating can comprise a calcium source which is insoluble calcium silicate, present as the composite material calcium oxide-silica (CaO-SiO₂) as described in commonly-owned applications, World Application Nos. 2008/015117 and 2008/068248.

When a calcium silicate composite material is employed as coating, the ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise monocalcium silicate, bi-calcium silicate, or tri-calcium silicate whereby ratios of calcium to silicon (Ca:Si) should be understood to be atom ratios.

The preferred calcium source employed in this invention to generate second component coating on the composite particle active may be in a crystalline or amorphous state. In an often preferred embodiment, the calcium source for coating is in a mesoporous state, i.e. the source is a material having pores with diameters from 1 nm to 1 micron. Mesoporous calcium silicate (MCS) is often preferred.

The MCS which may be used in second component coating in this invention can be made by combining a calcium salt (e.g., calcium chloride, calcium carbonate, calcium hydroxide), a silica precursor like silicate (e.g., sodium silicate, potassium silicate, tetraethyl orthosilicate or tetraethylsilicate) and a structure-directing agent to yield a solid suitable for calcinating. A more detailed description of the process that may be conducted to make the MCS suitable for use in this invention is described in the aforementioned commonly-owned application, Publication No. WO 2008/015117.

In an often desired embodiment, coating may be formed from CaO-SiO₂.

In still another often preferred embodiment, oral care compositions comprising the composite particle active of this invention further comprise a phosphate source. The phosphate source that may be used in this invention is limited only to the extent that the same may be used in a composition suitable for use in the mouth. Illustrative examples of the types of phosphate source suitable for use in this invention and added along with the composite particle actives described include monosodium phosphate, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate, mixtures thereof or the like. The phosphate source is preferably one which is water soluble.

When used, the phosphate source typically makes up from 0.5 to 22%, and preferably, from 2 to 18%, and most preferably, from 4 to 16% by weight of the oral care composition, based on total weight of the oral care composition and including all ranges subsumed therein. In a preferred embodiment, the phosphate source used is one which results in an oral care composition (i.e., combined composition if a dual phase product) having a pH from 5.5 to 8, preferably from 6 to 7.5, and most preferably, about neutral. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

Oral care compositions for benefitting teeth and comprising the composite particle active of this invention may comprise optional ingredients which are common in the art. These ingredients include:
▪ antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds such as 2,2' methylenebis-(4-chloro-6-bromophenol);
▪ anti-inflammatory agents such as ibuprofen, flurbiprofen, aspirin, indomethacin, etc.;
▪ anti-caries agents such as sodium trimetaphosphate and casein;
▪ plaque buffers such as urea, calcium lactate, calcium glycerophosphate and strontium polyacrylates;
▪ vitamins such as Vitamins A, C and E;
▪ plant extracts;
▪ desensitizing agents, e.g. potassium citrate, potassium chloride, potassium tartrate, potassium bicarbonate, potassium oxalate, and potassium nitrate;
▪ anti-calculus agents, e.g. alkali-metal pyrophosphates, hypophosphite-containing polymers, organic phosphonates and phosphocitrates, etc.;
▪ biomolecules, e.g. bacteriocins, antibodies, enzymes, etc.
▪ flavors, e.g., peppermint and spearmint oils;
▪ proteinaceous materials such as collagen;
▪ preservatives;
▪ opacifying agents;
▪ colouring agents like FD&C blue, yellow and/or red dyes/colorants;
▪ pH-adjusting agents;
▪ sweetening agents;
▪ surfactants, such as anionic, nonionic, cationic and zwitterionic or amphoteric surfactants (e.g., sodium lauryl sulfate, sodium dodecylbenzene sulfonate);
▪ particulate abrasive materials such as abrasive silicas, aluminas, calcium carbonates, zirconium silicate, polymethylmethacrylate, dicalciumphosphates, calcium pyrophosphates, hydroxyapatites, trimetaphosphates, insoluble hexametaphosphates as well as agglomerated particulate abrasive materials;
▪ fluoride sources like sodium fluoride, stannous fluoride, sodium monofluorophosphate, zinc ammonium fluoride, tin ammonium fluoride, calcium fluoride, cobalt ammonium fluoride or mixtures thereof;
▪ polymeric compounds which can enhance the delivery of active ingredients such as antimicrobial agents can also be included. Examples of such polymers are copolymers of polyvinylmethylether with maleic anhydride and other similar delivery enhancing polymers, e.g., those described in DE-A03,942,643;
▪ buffers and salts to buffer the pH and ionic strength of the oral care compositions; and
▪ other optional ingredients that may be included are, e.g., bleaching agents such as peroxy compounds, e.g., potassium peroxydiphosphate, effervescing systems such as sodium bicarbonate/citric acid systems, color change systems, and the like.

Such ingredients typically and collectively make-up less than 20% by weight of the oral care composition comprising the composite particle active of this invention, and preferably, from 0.0 to 15% by weight, and most preferably, from 0.01 to 12% by weight of the oral care composition, including all ranges subsumed therein.

Thickener may also be used in this invention and is limited only to the extent that the same may be added to a composition suitable for use in the mouth along with the composite particle actives of this invention. Illustrative examples of the types of thickeners that may be used in this invention include, sodium carboxymethyl cellulose, hydroxyl ethyl cellulose, methyl cellulose, ethyl cellulose, gum tragacanth, gum Arabic, gum karaya, sodium alginate, carrageenan, guar, xanthan gum, Irish moss, starch, modified starch, silica based thickeners including silica aerogels, magnesium aluminum silicate (i.e., Veegum) Carbomers (cross-linked acrylates) and mixtures thereof.

Typically, sodium carboxymethyl cellulose and/or a Carbomer is/are preferred. When a Carbomer is employed, those having a molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Carbopol^{®} 980. It has been described as a high molecular weight and cross-linked polyacrylic acid and identified via CAS number 9063-87-0. The same is available commercially from Lubrizol Advanced Materials, Inc.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickener typically makes up from 0.01 to about 10%, and preferably, from 0.1 to 8%, and most preferably, from 1.5 to 6% by weight of the oral care composition, based on total weight of the oral care composition and including all ranges subsumed therein.

Suitable carriers that may be employed in this invention are, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. The carriers should, in any case, be substantially free of water, and preferably, anhydrous if a single phase product comprising phosphate as an additive and composite particle active are desired. The carrier can, for example, be used in solid form, but glycerin is often the preferred carrier or humectant in single phase products.

The carrier is used to take the balance of the single phase compositions up to 100%, and the same may be present in the range from 10 to 90% by weight of the single phase oral care composition. Preferably, carrier makes up from 25 to 80%, and most preferably, from 45 to 70% by weight of the single phase oral care composition, based on total weight of the single phase oral care composition and including all ranges subsumed therein.

In the event an aqueous oral care product with a phosphate source is desired, a dual phase oral care composition is recommended to prevent interaction (prior to use) between the phosphate and second component coating of the composite particle active.

When a dual phase oral care composition is desired, water may act as a carrier (along with thickeners and/or additional carriers herein described) and make up the balance of each composition in the dual phase product wherein a first composition will comprise composite particle active and a second composition will comprise the added phosphate source. Within the dual phase product, the first and second compositions should not come into contact with each other until dispensed for use by the consumer in his or her mouth. When a dual phase oral care composition is used, the weight percents described herein are meant to characterize the oral care composition after the first and second compositions have been combined. The delivery of each composition (in the event a dual composition product is desired) may be sequential or simultaneous, but preferably, simultaneous. In a preferred embodiment, each composition within the dual phase oral care composition comprises less than 35% by weight water, and most preferably, from 15 to 25% by weight water, based on total weight of each composition and including all ranges subsumed therein.

One or preferably both of the compositions within the dual phase oral care composition may be applied to the teeth, with treatment of the teeth involving mixing of the compositions. Whether single or dual phase, the compositions are preferably left on the teeth following application. Following such application, the oral care compositions of this invention should typically be left on the teeth for 5 seconds to 10 hours and more typically from 35 seconds to 30 minutes. The application may be carried out daily. If a dual composition is employed, the same may be applied from independent compartments at a dual compartment tube or from independent phases of a product contained within a single container which is typically a tube.

In certain embodiments, in particular those involving a gel composition, the means of delivery may optionally involve a tape, in particular an adhesive tape strip, onto which the compositions of this invention are applied prior to the strip being placed in contact with the teeth. When using such a means of delivery, the compositions can be held in close contact with the surface of the teeth, facilitating a high concentration of composite particle active and phosphate close to the surface of the teeth.

When a gel is desired, the same typically comprises a polymeric matrix, and is more typically a hydrogel. Excluding any water present, the polymeric matrix is typically present at from 1 to 25% by weight of the composition of which it is a part.

Monomers used to prepare hydrogels may be selected from, for example, vinyl alcohol and acrylate, in particular sodium acrylate. Other monomers comprising an abundance of hydrophilic groups may also be used.

Often preferred hydrogels comprise a polysaccharide, polyacrylamide, polyacrylic acid, or a mixture thereof.

Suitable polysaccharides may be storage polysaccharides, such as starch or glycogen, or structural polysaccharides, such as cellulose or chitin.

Illustrative polysaccharides which may be used include those having saccharide units selected from one or more of the following: isomaltose, glucose, fructose, galactose, xylose, mannose, sorbose, arabinose, rhamnose, fucose, maltose, sucrose, lactose, maltulose, ribose, lyxose, allose, altrose, gulose, idose, talose, trehalose, nigerose, kojibiose, and lactulose.

Often preferred hydrogels comprise at least one polysaccharide selected from the group consisting of: tamarind gum, guar gum, locust bean gum, Tara, Fenugreek, Aloe, Chia, Flaxseed, Psyllium seed, quince seed, xanthan, gellan, welan, rhamsan, dextran, curdlan, pullulan, scleroglucan, schizophyllan, chitin, hydroxyalkyl cellulose, arabinan, de-branched arabinan, arabinoxylan, glactan, pectic galactan, galactomannan, glucomannan, lichenan, mannan, pachyman, rhamnogalacturonan, acacia gum, agar, alginates, carrageenan, chitosan, clavan, hyaluronic acid, heparin, inulin, cellodextrins, cellulose, and cellulose derivatives.

Especially preferred hydrogels can comprise polysaccharides like those selected from the group consisting of: sodium alginate, hydroxypropyl alginate, gum carrageenan, gum arabic, guar gum, karaya gum, chitosan and pectin.

Compositions comprising the composite particle actives of this invention (whether single or dual phase) are prepared by conventional methods of making oral care compositions. Such methods include mixing the ingredients under moderate shear and atmospheric pressure. The compositions are desired for use in the mouth, and preferably, are of the form that may be brushed onto teeth with a toothbrush. Unexpectedly, the oral care compositions of this invention result in excellent remineralization of teeth (i.e., new hydroxyapatite formation) and teeth whitening (which may be immediate and provided for by the core of the composite particle active) as a result of composite particle active adhering to enamel and/or dentin of teeth. Moreover, subsequent to using the compositions of this invention, teeth are unexpectedly less sensitive, and shinier, the same also being a direct result of composite particle active adhering to the enamel and/or dentin of teeth.

The composite particle active of this invention may be prepared by creating a slurry comprising core material and solvent (e.g., low molecular weight ketones like acetone, low molecular weight alcohols like C₃₋₆ alkanols, water or mixtures thereof), the slurry comprising 3 to 60%, and preferably, 5 to 30%, and most preferably, 5 to 20% by weight core material based on total weight of the slurry. Water is typically the preferred solvent. The core material (like titanium oxide or mica) slurry should be heated from 25 to 95°C, and preferably, from 55 to 95°C, and most preferably, from 70 to 90°C, including all ranges subsumed therein. While heating, precursor for anionic portion of coating (like sodium silicate or silica) should then be added to the heated slurry to provide for coating on core material. The curing of the anionic portion (i.e., anionic precursor portion) within the slurry should last from 0.5 hours to 3 hours, and preferably, from 0.5 hours to 1 hour at an alkaline pH (preferably 8.5 to pH 11, and most preferably, 9 to pH 10.5). Further but preferred curing of the slurry can be achieved under acidic conditions (i.e., preferably 3 to pH 5), for an additional 0.5 to 2 hours when the core material is not soluble in the slurry under such acidic conditions. Subsequent to curing the anionic portion of coating, chemical metal material suitable to supply cations, like calcium ions, should then be added to the resulting cured core slurry. The resulting mixture with cations should be cured from 0.5 hours to 24 hours, and preferably, from 0.5 hours to 2 hours at a pH from 9 to 13 and at temperatures within the ranges for curing core. In an often preferred step, anionic precursor reagents like sodium alginate and/or sodium sulfate can further be added for *in situ* generation of coating material within the slurry. Slurry pH may be adjusted with conventional ingredients like NaOH or HCL. Mixing of ingredients may be achieved with conventional mixing apparatus. Often, however, a pipeline reactor having a zone of turbulence is often preferred. Conventional catalysts or structure directing agents may be used to generate composite particle. These agents typically make up from 0.01 to 3% by weight of the slurry when they are used. An often preferred agent is a cetyltrialkyl ammonium halide like cetyltrimethyl ammonium bromide and the like. In an especially preferred embodiment, silica is used in the core material and solvent slurry for coating manufacture. The resulting particles may be cleaned by conventional techniques which include filtration and/or centrifugation and subsequently dried. The desired composite particles of this invention may be recovered by drying such resulting particles where drying may be accelerated with heat and usually takes about 10 minutes to two (2) hours when heating to a temperature in the range from 65°C to 1000°C.

When the oral care composition of this invention is a toothpaste or gel, the same typically has a viscosity from about 50,000 to 180,000 centipoise, and preferably, from 60,000 to 170,000 centipoise, and most preferably, from 65,000 to 165,000 centipoise, taken at room temperature with a Brookfield Viscometer, Spindle No. 4.

In toothpaste or gel form, the composition may be packaged in a conventional plastic laminate, metal tube or a single compartment dispenser. The same may be applied to dental surfaces by any physical means, such as a toothbrush, fingertip or by an applicator directly to the sensitive area. Solid dosage form types include pastilles, lozenges, chewing gums, tablets, mouthstrips, balms, and the like. These may be contained in conventional packaging desirable for consumer use.

The following examples are provided to facilitate an understanding of the present invention. The examples are not provided to limit the scope of the claims.

### Example 1

A dual phase aqueous-based oral care composition for benefitting teeth and consistent with this invention (i.e., comprising composite particle active) was made by mixing the ingredients below under conditions of moderate shear, atmospheric pressure and ambient temperature. The compositions made were suitable for use with a toothbrush, and when combined about equally were not gritty and resulted in an excellent ribbon when applied to a toothbrush. The combined compositions, which resulted in the oral care composition of this invention, had consumer acceptable taste characteristics.

### Calcium Comprising Phase:

| Ingredients | Percent by Weight |
|---|---|
| Sorbitol (70%) | 20.0 |
| Water | balance |
| Preservative | 1.0 |
| Sodium monofluorophosphate | 1.1 |
| Sweetener (artificial) | 0.2 |
| Calcium silicate* | 20.0 |
| Abrasive silica | 6.0 |
| Thickening silica (fumed silica) | 2.5 |
| Calcium silicate coated titanium dioxide** | 4.00 |
| Flavor | 0.9 |
| Sodium carboxymethyl cellulose | 0.6 |
| Sodium lauryl sulphate (30%) | 6.60 |

| | |
|---|---|
| * as supplied by Ineos Silicas, Ltd. ** composite particle active, coating ~ 10 nm; particle diameter ~ 100 to 300 nm | |

### Phosphate Comprising Phase:

| Ingredients | Percent by Weight |
|---|---|
| Sorbitol (70%) | 55.0 |
| Trisodium phosphate | 7.6 |
| Water | Balance |
| Polyethylene glycol (1500) | 2.0 |
| Sodium monofluorophosphate | 1.1 |
| Sweetener (artificial) | 0.27 |
| Color | 0.002 |
| Abrasive silica | 12.0 |
| Monosodium phosphate | 6.4 |
| Thickening silica (fumed silica) | 3.5 |
| Sodium lauryl sulphate 30% | 6.6 |
| Flavour | 1.2 |
| Sodium carboxymethyl cellulose | 0.5 |

### Example 2

Enamel and dentin blocks (3 cm²) were polished for at about 5 minutes using silicone carbide abrasive paper (1200 grit). The dentin blocks were acid etched for 60 seconds using 36% phosphoric acid, followed by a water wash. The blocks were purchased from a commercial supplier.

A slurry comprising 20% by weight calcium silicate and 4% by weight composite particle active (similar to the one described in Example 1) were added to a water balance to make a slurry. A sodium phosphate slurry was made by adding 20% by weight sodium phosphate to a water balance. The resulting slurries were homogenized for about 10 minutes using conventional stirring apparatus in order to yield homogeneous compositions.

The homogenized slurries were poured into petri dishes. Enough was used to cover the surface of the blocks which were placed in the dishes. The blocks were brushed with a toothbrush for 1 minute and incubated in the slurry for one minute. The blocks were subsequently washed with distilled water three times and further incubated in simulated oral cavity fluid for at about 2 hours in a 37°C water bath. Treatment for each block was repeated 14 times.

### Results of Treatment - Enamel Blocks

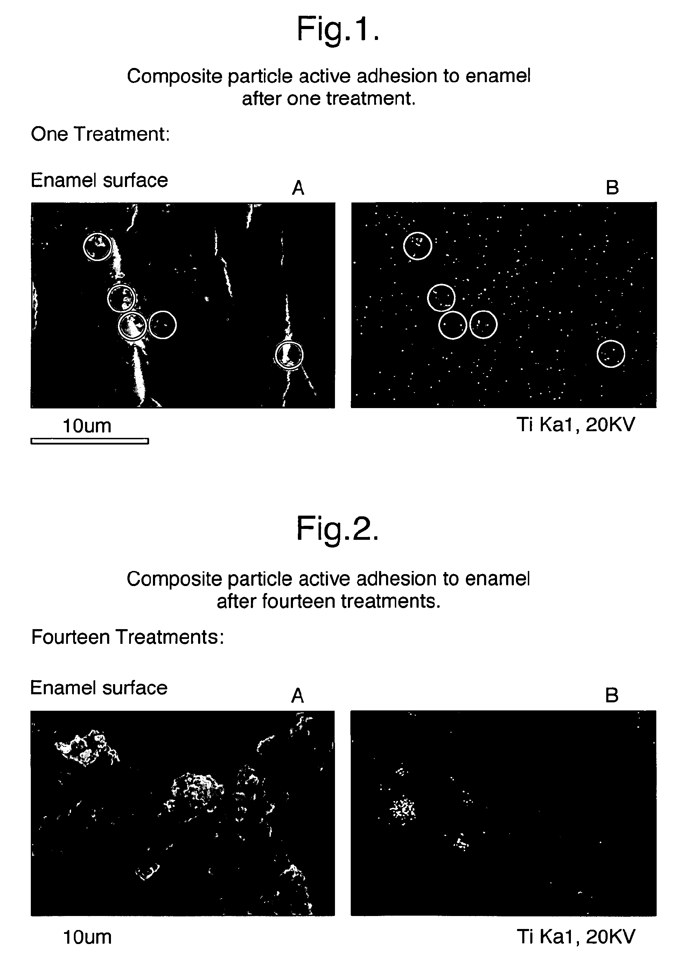

Figure 1A shows a scanning electron microscopy image of the enamel block after one treatment. Composite particle active (circled) surprisingly is shown adhering to the surface of the enamel block after one treatment.

Figure 1B shows an energy dispersive x-ray image (elemental maps, Ti Ka1, 20KV) of the same block. Composite particle active is shown as bright dots (circled) surprisingly adhering well to the enamel block after one treatment.

Figure 2A shows a scanning electron microscopy image of the enamel block after 14 treatments and Figure 2B shows an energy dispersive x-ray image of the same block having received fourteen treatments. The results unexpectedly demonstrate excellent adhesion of the composite particle active to the enamel surface.

### Results of Treatment - Dentin Blocks

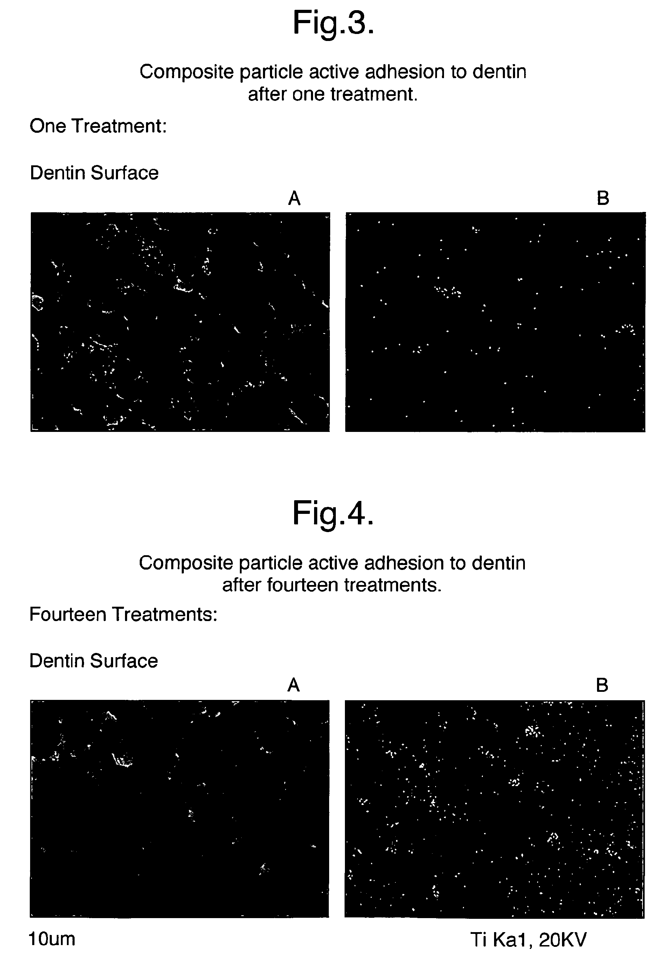

Figure 3A shows a scanning electron microscopy image of the dentin block after one treatment. Composite particle active surprisingly is shown adhering to the surface of the dentin.

Figure 3B shows an energy dispersive x-ray image (elemental maps, Ti Ka1 20KV) of the same block. Composite particle active surprisingly is shown adhering to the dentin block.

Figure 4A shows a scanning electron microscopy image of the dentin block after 14 treatments and Figure 4B shows an energy dispersive x-ray image of the same block having received the fourteen treatments. The results unexpectedly demonstrate excellent adhesion of the composite particle active to the dentin surface.

The unexpected adhesion of the active compositions of this invention to tooth enamel and dentin inevitably will result in tooth remineralization, desensitizing and shine.

### Example 3

To investigate the whitening effect of the illustrative dual phase oral care composition of Example 1, the following *in vitro* test was performed.

Human teeth were cleaned and separated into two groups (N=8). While treating, the composition of Example 1 was applied to the surface of teeth (Group I) with cotton buds. After 30 minutes, the treated teeth were brushed with a slurry of commercially available toothpaste and water (e.g., fluoride comprising toothpaste, 1 % sodium fluoride : water = 1:2) for one (1) minute to clean the treated surface. The cleaned teeth were incubated in simulated oral fluid for about 2 hours. The teeth were subject to 14 cycles of the above treatment. Simulated oral care fluid was made by combining the following:

### Simulated Oral Fluid

| Ingredient | Amount |
|---|---|
| NaCl | 16.07g |
| NaHCO₃ | 0.7g |
| KCI | 0.448g |
| K₂HPO₃*H₂O | 3.27g |
| MgCl₂*6H₂O | 0.622g |
| 1M HCl | 40ml |
| CaCl₂ | 0.1998g |
| Na₂SO₄ | 0.1434g |
| Buffer | Adjust pH to 7.0 |
| Water | Balance to 2L |

The treatments to the teeth of Group II were the same as that for Group I except that only the commercially available toothpaste was used.

Changes in the whiteness between pre- and post-treatment were obtained using a Konica Minolta - 2600D colour analyzer.

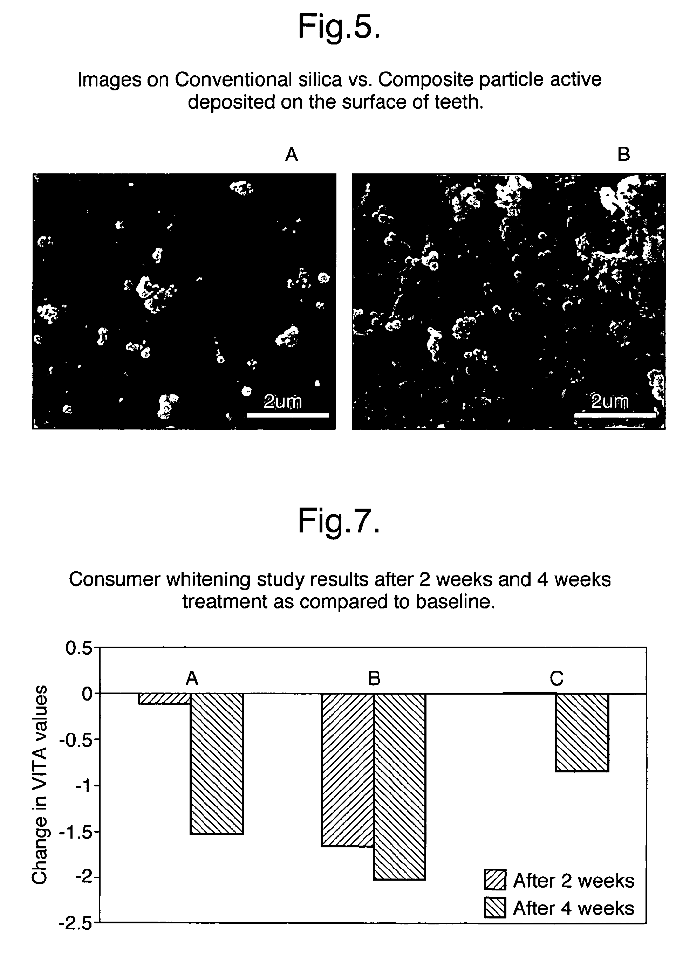

The results, unexpectedly demonstrate that teeth treated with the composition of this invention had an excellent whitening index of about 4.80 whereby conventionally available product had a whitening index of only about -0.91. Moreover, Figure 5 shows scanning electron microscopy images of teeth treated with conventional silica comprising toothpaste (5A) and calcium silicate coated silica (composite particle active) (5B) consistent with this invention. Surprisingly, as may be observed in the images, significantly more particles (especially particles/agglomerates at circa 2 µm diameter in size) deposited on the surface of teeth when made consistent with this invention.

### Example 4

A slurry of 10 weight % titanium dioxide and water was prepared by stirring under atmospheric conditions. The slurry was heated to about 90°C for 30 to 35 minutes. A 1M sodium silicate solution was added to the heated slurry (S₁O₂/T₁O₂, 10 weight %). Hydrochloric acid (0.5 M) was added to buffer the pH of the slurry from 9 to 10.5 for a one (1) hour period. Heat was removed so that the resulting slurry was cooled to around 70°C. To the cooled slurry was added a 1 M CaCl₂ solution (CaCl₂/SiO₂, 1 M, 1 M). A 1 M sodium hydroxide solution was also added to the resulting slurry to buffer pH in the range of 10.5 to 12 for a one (1) hour period. The resulting slurry was cleaned via centrifugation and dried at 90°C for about 45 minutes. The resulting particles were composite particle actives consistent with this invention.

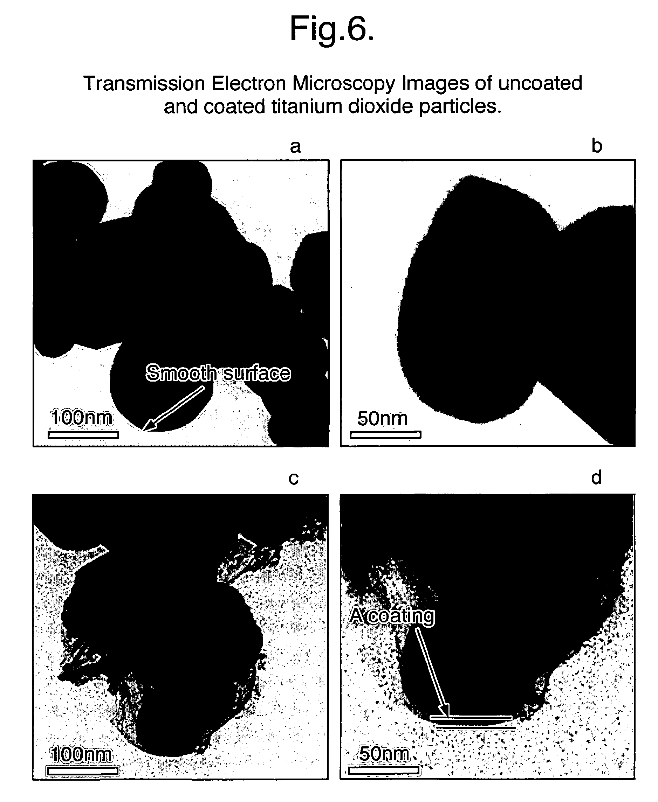

Figure 6 shows Transmission Electron Microscopy Images of uncoated titanium dioxide 6a and 6b and calcium silicate coated titanium dioxide made consistent with this invention (6c and 6d). The second component coating was homogeneous in that all of the titanium dioxide core was coated and the calcium silicate coating was about 10 nm thick.

### Example 5

A composite particle active similar to the one described in Example 4 was made except that zinc oxide was used in lieu of titanium dioxide. Transmission Electron Microscopy Images revealed the successful calcium silicate coating of zinc oxide core when performing the method of this invention.

### Example 6

To investigate the whitening effect of the illustrative dual phase oral care composition of Example 1, the following consumer study was performed.

Selected subjects, having an initial teeth color range higher than 3M1 as determined by the tooth guide 3D-Master from Vita Zahnfabrik, were divided into about three equal groups, (N=55) and groups A, B and C. All subjects were asked to brush their teeth twice a day. Each group was given a different toothpaste. Group B subjects were given the dual phase oral care composition of Example 1 and consistent with this invention; Group A subjects were given a dual phase composition similar to the one described in Example 1 except that 30% by weight calcium silicate was used and no composite particle active was employed. Group C (the control group) subjects brushed teeth with commercially available toothpaste compositions deplete of active associated with tooth remineralization.

Changes in the whiteness between pre- and post- treatment (after 2 weeks and 4 weeks brushing) were obtained using the aforementioned VITA shade tooth guide 3D-Master from Vita Zahnfabrik.

The results presented in Figure 7, unexpectedly, demonstrate that the teeth of subjects in Group B obtained a significant decrease in VITA value after 4 weeks treatment, and a detectable decrease after only 2 weeks of treatment. Teeth from subjects in Group A obtained a decrease in VITA value but the decrease was essentially not detectable prior to 4 weeks of treatment. The teeth of subjects from control Group C displayed no significant change in VITA value after 4 weeks treatment.

The results demonstrate significant reduction in VITA values for teeth of subjects using compositions made consistent with this invention whereby the whitening effect to teeth of subjects using an oral care composition consistent with this invention were, surprisingly, significantly better when compared to teeth treated with compositions deplete of composite particle active. Figure 7 shows the change in VITA values from a baseline after 2 weeks and 4 weeks for the groups tested. The unexpected and superior whitening results obtained when using oral care compositions consistent with this invention is shown for Group B.

## Claims

1. A composite particle active comprising:
a) a first component core for improving an oral care characteristic wherein the first component core comprises silica, titanium dioxide, zinc oxide, mica, calcium carbonate, barium sulfate, or a mixture thereof; and
b) a second component coating that interacts with phosphate ions to produce a calcium phosphate *in situ* reaction product that adheres to tooth enamel, dentin or both and that is a precursor for hydroxyapatite formation and wherein the second component coating comprises calcium gluconate, calcium oxide, calcium lactate, calcium hydroxide, calcium sulfate, calcium carboxymethyl cellulose, calcium alginate, calcium salts of citric acid, calcium silicate or a mixture thereof;
wherein at least 70 to 100% of the outer surface area of the first component core is coated with second component coating.

2. The composite particle active according to claim 1 wherein the composite particle active has a diameter of less than 50 microns.

3. The composite particle active according to any of the preceding claims wherein the core of the composite particle active makes up from 3 to 98% by weight of the composite particle active.

4. The composite particle active according to any of the preceding claims wherein the core comprises at least 50% by weight titanium dioxide or zinc oxide based on total weight of the first component core.

5. The composite particle active according to claim 4 wherein the first component core comprises at least 50% by weight titanium dioxide and the second component coating comprises at least 50% by weight calcium element.

6. The composite particle active according to claim 1 wherein the second component coating comprises calcium silicate which is CaSiO₃, CaO-SiO₂ or a mixture thereof.

7. The composite particle active according to any of the preceding claims wherein the composite particle active comprises a diameter from 10 nm to less than 10 microns.

8. The composite particle active according to claim 7 wherein at least 40% of the diameter of the composite particle active is the result of core.

9. The composite particle active according to claim 8 wherein from 75 to 99.5% of the diameter of the composite particle active is the result of core.

10. The composite particle active according to any of the preceding claims wherein the second component coating comprises calcium silicate having a calcium to silica ratio from 1:10 to 3:1.

11. The composite particle active according to any of the preceding claims wherein the second component coating comprises mesoporous calcium silicate.

12. A method for making the composite particle active of claim 1 comprising the steps of:
a) mixing core material with solvent to product a core slurry;
b) heating the core slurry from 25°C to 95°C for 0.5 to 3 hours;
c) adding reagent suitable for *in situ* generation of coating material to produce a core and coating material slurry; and
d) drying the core and coating material slurry to recover the composite particle active
wherein the core slurry is first heated under alkaline conditions and subsequently acidic conditions if the core material is insoluble under acidic conditions.

13. The method according to claim 12 wherein the reagent suitable for *in situ* generation of coating material comprises silica, sodium silicate, potassium silicate, sodium alginate, sodium sulfate, tetraethyl orthosilicate, or a mixture thereof.

14. The method according to claim 12 wherein the reagent suitable for *in situ* generation of coating material comprises calcium silicate, calcium phosphate, calcium gluconate, calcium oxide, potassium chloride, calcium lactate, calcium carbonate, calcium hydroxide, calcium sulfate, calcium carboxymethyl cellulose, Calcium chloride, magnesium chloride, aluminum chloride, calcium alginate, calcium salts of citric acid, mixtures thereof or the like.

## Patentansprüche

1. Zusammengesetzter Partikelwirkstoff, umfassend
a) einen Kern eines ersten Bestandteils zur Verbesserung eines charakteristischen Mundpflegemerkmals, wobei der Kern des ersten Bestandteils Siliziumdioxid, Titandioxid, Zinkoxid, Glimmer, Calciumcarbonat, Bariumsulfat oder eine Mischung davon umfasst, und
b) eine Beschichtung eines zweiten Bestandteils, der mit Phosphationen in Wechselwirkung tritt, um durch eine *in situ*-Reaktion ein Calciumphosphatprodukt herzustellen, das am Zahnschmelz oder dem Dentin oder beidem haftet und das eine Vorstufe für die Bildung von Hydroxyapatit darstellt und wobei die Beschichtung des zweiten Bestandteils Calciumglukonat, Calciumoxid, Calciumlaktat, Calciumhydroxid, Calciumsulfat, Calciumhydroxymethylcellulose, Calciumalginat, Calciumsalze von Zitronensäure, Calciumsilikat oder eine Mischung davon umfasst,
wobei mindestens 70 bis 100% des äußeren Oberflächenbereichs des Kerns des ersten Bestandteils mit der Beschichtung des zweiten Bestandteils beschichtet ist.

2. Zusammengesetzter Partikelwirkstoff nach Anspruch 1, wobei der zusammengesetzte Partikelwirkstoff einen Durchmesser von weniger als 50 Mikrometer aufweist.

3. Zusammengesetzter Partikelwirkstoff nach irgendeinem der vorhergehenden Ansprüche, wobei der Kern des zusammengesetzten Partikelwirkstoffs 3 bis 98 Gewichts-% des zusammengesetzten Partikelwirkstoffs ausmacht.

4. Zusammengesetzter Partikelwirkstoff nach irgendeinem der vorhergehenden Ansprüche, wobei der Kern mindestens 50 Gewichts-% Titandioxid oder Zinkoxid, beruhend auf dem Gesamtgewicht des Kerns des ersten Bestandteils, umfasst.

5. Zusammengesetzter Partikelwirkstoff nach Anspruch 4, wobei der Kern des ersten Bestandteils mindestens 50 Gewichts-% Titandioxid und die Beschichtung des zweiten Bestandteils mindestens 50 Gewichts-% Calcium-Element umfasst.

6. Zusammengesetzter Partikelwirkstoff nach Anspruch 1, wobei die Beschichtung des zweiten Bestandteils Calciumsilikat, welches CaSiO₃, CaO-SiO₂ oder eine Mischung davon darstellt, umfasst.

7. Zusammengesetzter Partikelwirkstoff nach irgendeinem der vorhergehenden Ansprüche, wobei der zusammengesetzte Partikelwirkstoff einen Durchmesser von 10 nm bis weniger als 10 Mikrometer umfasst.

8. Zusammengesetzter Partikelwirkstoff nach Anspruch 7, wobei mindestens 40% des Durchmessers des zusammengesetzten Partikelwirkstoffs das Resultat des Kerns darstellen.

9. Zusammengesetzter Partikelwirkstoff nach Anspruch 8, wobei 75 bis 99,5% des Durchmessers des zusammengesetzten Partikelwirkstoffs das Resultat des Kerns darstellen.

10. Zusammengesetzter Partikelwirkstoff nach irgendeinem der vorhergehenden Ansprüche, wobei die Beschichtung des zweiten Bestandteils Calciumsilikat mit einem Verhältnis Calcium-zu-Siliziumdioxid von 1:10 bis 3:1 umfasst.

11. Zusammengesetzter Partikelwirkstoff nach irgendeinem der vorhergehenden Ansprüche, wobei die Beschichtung des zweiten Bestandteils mesoporöses Calciumsilikat umfasst.

12. Verfahren zur Herstellung des zusammengesetzten Partikelwirkstoffs nach Anspruch 1, umfassend die Schritte:
a) Mischen von Kernmaterial mit Lösungsmittel, um eine Kernaufschlämmung herzustellen,
b) Erhitzen der Kernaufschlämmung von 25°C bis 95°C für 0,5 bis 3 Stunden,
c) Zugabe von Reagenz, das zur *in situ*-Bildung von Beschichtungsmaterial geeignet ist, um eine Kern- und Beschichtungsmaterial-Aufschlämmung herzustellen, und
d) Trocknen der Kern- und Beschichtungsmaterial-Aufschlämmung, um den zusammengesetzten Partikelwirkstoff zu gewinnen,
wobei die Kernaufschlämmung zuerst unter alkalischen Bedingungen und anschließend unter sauren Bedingungen, wenn das Kernmaterial unter sauren Bedingungen unlöslich ist, erhitzt wird.

13. Verfahren nach Anspruch 12, wobei das Reagenz, das zu einer *in situ*-Bildung von Beschichtungsmaterial geeignet ist, Siliziumdioxid, Natriumsilikat, Kaliumsilikat, Natriumalginat, Natriumsulfat, Tetraethylorthosilikat oder eine Mischung davon umfasst.

14. Verfahren nach Anspruch 12, wobei das Reagenz, das zur *in situ*-Bildung von Beschichtungsmaterial geeignet ist, Calciumsilikat, Calciumphosphat, Calciumgluconat, Calciumoxid, Kaliumchlorid, Calciumlaktat, Calciumcarbonat, Calciumhydroxid, Calciumsulfat, Calciumcarboxymethylcellulose, Calciumchlorid, Magnesiumchlorid, Aluminiumchlorid, Calciumalginat, Calciumsalze von Zitronensäure, Mischungen davon oder dergleichen umfasst.

## Revendications

1. Matière active de particule composite comprenant :
a) un noyau de premier constituant pour améliorer une caractéristique de soin oral où le noyau de premier constituant comprend de la silice, du dioxyde de titane, de l'oxyde de zinc, du mica, du carbonate de calcium, du sulfate de baryum, ou un mélange de ceux-ci ; et
b) un revêtement de second constituant qui interagit avec des ions phosphate pour produire un produit de réaction *in situ* de phosphate de calcium qui adhère à l'émail des dents, la dentine ou les deux et qui est un précurseur pour la formation d'hydroxyapatite et où le revêtement de second constituant comprend du gluconate de calcium, oxyde de calcium, lactate de calcium, hydroxyde de calcium, sulfate de calcium, calcium carboxyméthylcellulose, alginate de calcium, sels de calcium d'acide citrique, silicate de calcium ou un mélange de ceux-ci ;
où d'au moins 70 à 100 % de la surface externe du noyau de premier constituant sont revêtus avec le revêtement de second constituant.

2. Matière active de particule composite selon la revendication 1, dans laquelle la matière active de particule composite présente un diamètre inférieur à 50 microns.

3. Matière active de particule composite selon l'une quelconque des revendications précédentes, dans laquelle le noyau de la matière active de particule composite constitue de 3 à 98 % en masse de la matière active de particule composite.

4. Matière active de particule composite selon l'une quelconque des revendications précédentes, dans laquelle le noyau comprend au moins 50 % en masse de dioxyde de titane et d'oxyde de zinc rapporté à la masse totale du noyau de premier constituant.

5. Matière active de particule composite selon la revendication 4, dans laquelle le noyau de premier constituant comprend au moins 50 % en masse de dioxyde de titane et le revêtement de second constituant comprend au moins 50 % en masse d'élément de calcium.

6. Matière active de particule composite selon la revendication 1, dans laquelle le revêtement de second constituant comprend du silicate de calcium qui est CaSiO₂, CaO-SiO₂ ou un mélange de ceux-ci.

7. Matière active de particule composite selon l'une quelconque des revendications précédentes, dans laquelle la matière active de particule composite comprend un diamètre de 10 nm à moins de 10 microns.

8. Matière active de particule composite selon la revendication 7, dans laquelle au moins 40 % du diamètre de la matière active de particule composite résultent du noyau.

9. Matière active de particule composite selon la revendication 8, dans laquelle de 75 à 99,5 % du diamètre de la matière active de particule composite résultent du noyau.

10. Matière active de particule composite selon l'une quelconque des revendications précédentes, dans laquelle le revêtement de second constituant comprend du silicate de calcium ayant un rapport calcium à silice de 1:10 à 3:1.

11. Matière active de particule composite selon l'une quelconque des revendications précédentes, dans laquelle le revêtement de second constituant comprend du silicate de calcium mésoporeux.

12. Procédé de fabrication de la matière active de particule composite selon la revendication 1 comprenant les étapes de :
a) mélange de matériau de noyau avec un solvant pour produire une suspension de noyau ;
b) chauffage de la suspension de noyau de 25°C à 95°C pendant de 0,5 à 3 heures ;
c) addition de réactif approprié pour une production *in situ* de matériau de revêtement pour produire une suspension de noyau et de matériau de revêtement ; et
d) séchage de la suspension de noyau et de matériau de revêtement pour recouvrir la matière active de particule composite
dans lequel la suspension de noyau est dans un premier temps chauffée dans des conditions alcalines et ensuite dans des conditions acides si le matériau de noyau est insoluble dans des conditions acides.

13. Procédé selon la revendication 12, dans lequel le réactif approprié pour une production *in situ* de matériau de revêtement comprend de la silice, du silicate de sodium, du silicate de potassium, de l'alginate de sodium, du sulfate de sodium, de l'orthosilicate de tétraéthyle, ou un mélange de ceux-ci.

14. Procédé selon la revendication 12, dans lequel le réactif approprié pour une production *in situ* de matériau de revêtement comprend du silicate de calcium, phosphate de calcium, gluconate de calcium, oxyde de calcium, chlorure de potassium, lactate de calcium, carbonate de calcium, hydroxyde de calcium, sulfate de calcium, calcium carboxyméthylcellulose, chlorure de calcium, chlorure de magnésium, chlorure d'aluminium, alginate de calcium, sels de calcium d'acide citrique, mélanges de ceux-ci ou semblable.
